(19) Europäisches Patentamt / European Patent Office / Office européen des brevets

(11) EP 3 492 446 B1

(12) # EUROPÄISCHE PATENTSCHRIFT

(45) Veröffentlichungstag und Bekanntmachung des Hinweises auf die Patenterteilung:
**05.01.2022 Patentblatt 2022/01**

(21) Anmeldenummer: **18209299.9**

(22) Anmeldetag: **29.11.2018**

(51) Int Cl.:
*C07C 31/125* (2006.01) *C07C 29/82* (2006.01)
*C07C 29/80* (2006.01) *C07C 29/141* (2006.01)
*C07C 29/17* (2006.01) *C07C 45/50* (2006.01)
*C07C 47/02* (2006.01) *C07C 45/74* (2006.01)
*C07C 47/21* (2006.01)

(54) **VERFAHREN ZUR GEWINNUNG VON ALKOHOLEN AUS ALDEHYDEN**
METHOD FOR THE RECOVERY OF ALCOHOLS FROM ALDEHYDES
PROCÉDÉ D'OBTENTION D'ALCOOLS À PARTIR D'ALDÉHYDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.12.2017 EP 17204995**

(43) Veröffentlichungstag der Anmeldung:
**05.06.2019 Patentblatt 2019/23**

(73) Patentinhaber: **Evonik Operations GmbH**
**45128 Essen (DE)**

(72) Erfinder:
- **Fridag, Dirk**
**45721 Haltern am See (DE)**
- **Franke, Robert**
**45772 Marl (DE)**
- **Grass, Michael**
**45721 Haltern am See (DE)**
- **Boeck, Florian**
**40223 Düsseldorf (DE)**
- **Hecht, Corinna**
**45721 Haltern am See (DE)**
- **Dercks, Benedikt**
**44809 Bochum (DE)**
- **Lenz, Udo**
**45657 Recklinghausen (DE)**
- **Hiller, Christoph**
**48249 Dülmen (DE)**
- **Ehlers, Christoph**
**48153 Münster (DE)**
- **Münzner, Stefan**
**46282 Dorsten (DE)**

(74) Vertreter: **Evonik Patent Association**
**c/o Evonik Industries AG**
**IP Management**
**Bau 1042A/PB 15**
**Paul-Baumann-Straße 1**
**45772 Marl (DE)**

(56) Entgegenhaltungen:
**EP-A1- 2 796 440** **WO-A1-2008/065171**
**WO-A1-2017/080690** **DE-A1- 4 243 524**
**DE-A1-102009 001 594** **DE-A1-102009 045 718**
**DE-T2- 69 916 755** **US-A1- 2012 004 473**

- **?MER YILDIRIM ET AL: "Dividing wall columns in chemical process industry: A review on current activities", SEPARATION AND PURIFICATION TECHNOLOGY, Bd. 80, Nr. 3, 1. August 2011 (2011-08-01) , Seiten 403-417, XP055043075, ISSN: 1383-5866, DOI: 10.1016/j.seppur.2011.05.009**
- **PREMKUMAR R ET AL: "Retrofitting conventional column systems to dividing-Wall Columns", CHEMICAL ENGINEERING RESEARCH AND DESIGN, ELSEVIER, AMSTERDAM, NL, Bd. 87, Nr. 1, 1. Januar 2009 (2009-01-01) , Seiten 47-60, XP025803262, ISSN: 0263-8762, DOI: 10.1016/J.CHERD.2008.06.013 [gefunden am 2008-08-23]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 492 446 B1

## Beschreibung

Die vorliegende Erfindung betrifft ein Verfahren zur Herstellung von $C_n$- und $C_{2n}$-Alkoholen, wobei die Auftrennung der $C_n$-Alkohole und der $C_{2n}$-Alkohole durch mindestens ein Zweikolonnensystem oder durch mindestens eine Trennwandkolonne erfolgt.

Alkohole nehmen in der Industrie eine wichtige Rolle ein. Sie werden häufig als Zwischenprodukte für die Herstellung von Schmierölen, Fetten, Weichmachern, Pharmazeutika, Kosmetika und Geschmackstoffen verwendet. Direkten Einsatz finden Alkohole darüber hinaus als Lösemittel, Frostschutzmittel oder Treibstoffzusatz.

Weichmacher werden in großen Mengen zur Modifizierung der thermoplastischen Eigenschaften einer Vielzahl großtechnisch wichtiger Produkte, wie beispielsweise Kunststoffen, aber auch Lacken, Beschichtungsmitteln, Dichtungsmassen etc. eingesetzt. Eine wichtige Klasse von Weichmachern sind die Ester-Weichmacher, zu denen unter anderem Phthalsäureester, Trimellithsäureester, Phosphorsäureester etc. zählen. Die zur Herstellung der Ester-Weichmacher eingesetzten Alkohole werden allgemein als Weichmacheralkohole bezeichnet. Zur Herstellung von Ester-Weichmachern mit guten anwendungstechnischen Eigenschaften besteht ein Bedarf an Weichmacheralkoholen mit etwa 5 bis 12 Kohlenstoffatomen.

Bedingt durch die Phthalatdiskussion im Bereich der Weichmacher steigt die Nachfrage an neuen phthalatfreien Weichmachern. Dabei ist aber entscheidend, dass die jeweiligen Weichmacher im Hinblick auf die Anwendungen enge Vorgaben hinsichtlich ihrer Eigenschaften erfüllen müssen. Beispielhaft seien hier die Viskosität oder die Flüchtigkeit der Weichmacher genannt. Die Steuerung der wesentlichen Eigenschaften der Weichmacher hängt dabei weniger von der bei der Herstellung von Weichmachern üblicherweise eingesetzten Veresterungsreaktion sondern vielmehr von den eingesetzten Rohstoffen, insbesondere den eingesetzten Alkoholen ab. Wesentliche Faktoren sind hier beispielsweise der Anzahl der Kohlenstoffatome der eingesetzten Alkohole oder deren Isomerenverteilung. Hierbei bieten sich zum Beispiel die Alkohole mit 4, 5 oder 6 Kohlenstoffatomen an. Gleichzeitig ist es aber notwendig auch die beschriebenen C8-, C10- und C12-Alkohole zu produzieren.

Eine der bekanntesten Routen zu Alkoholen ist die Hydroformylierungsreaktion, bei der Alkene zu Aldehyden umgesetzt werden, die anschließend einer Hydrierung unterzogen werden, um zu den entsprechenden Alkoholen ($C_n$-Alkoholen) zu gelangen. Eine Ausnahme hierbei bildet die Hydroformylierung von Propen und unverzweigten Butenen. Hier werden die entstehenden Aldehyde meistens einem weiteren Reaktionsschritt, der Aldolisierung, unterzogen um zu langkettigen ungesättigten Aldehyden zu gelangen. Diese werden dann ebenfalls einer Hydrierung unterzogen und die erhaltenen längerkettigen Alkohole ($C_{2n}$-Alkohole) werden zum größten Teil in der Produktion von phthalathaltigen Weichmachern eingesetzt.

So beschreibt beispielsweise US 2014/350307 die Herstellung von Alkoholen aus Olefinen durch Hydroformylierung, Aldolkondensation und anschließende Hydrierung des Aldolkondensatgemisches.

US 2002/133047 beschreibt die gemeinsame Aldolkondensation von Butanalen und Pentanalen, um nach anschließender Hydrierung die isomeren Nonanole und Decanole zu erhalten.

US 6455743 beschreibt die Herstellung von Alkoholen durch Hydroformylierung eines Olefins, Aldolkondensation eines Teils des Aldehyds und anschließende Hydrierungsreaktion.

US 2010/048959 beschreibt ein Verfahren zur Herstellung von Hydroformylierungsprodukten aus Olefinen mit mindestens vier Kohlenstoffatomen und die anschließende Weiterverarbeitung zu Alkoholen, insbesondere 2-Propylheptanol.

DE 42 43 524 beschreibt ein Verfahren zur Herstellung von Pentanolen und Decanolen durch Aldolkondensation eines Gemisches aus n-Pentanal und 2-Methylbutanal, Hydrierung des Aldol-Produktes zu einem Gemisch enthaltend Pentanole und Decanole und destillative Auftrennung des Alkoholgemisches in Pentanole und Decanole.

Die Verfahren aus dem Stand der Technik haben den Nachteil, dass die Produktzusammensetzungen, insbesondere der finalen Alkohole, von den eingesetzten Eduktströmen, insbesondere von den eingesetzten Olefinen, abhängen. Eine gezielte Steuerung der Zusammensetzung der erhaltenen Alkohole ist, insbesondere im Hinblick auf Schwankungen bei den Eduktzusammensetzungen, auf diese Weise aber nicht möglich.

Daraus ergibt sich die Herausforderung, auf der einen Seite die Isomerenverteilung der herzustellenden Alkohole, auf der anderen Seite aber insbesondere auch das Verhältnis von $C_n$- zu $C_{2n}$-Alkoholen zu steuern.
Aufgabe der vorliegenden Erfindung ist die Bereitstellung eines Verfahrens, das die oben genannten Anforderungen erfüllt und die gezielte Steuerung der Zusammensetzung der erhaltenen Alkohole erlaubt.

Die Aufgabe der vorliegenden Erfindung wird durch ein Verfahren zur Herstellung von gesättigten $C_n$- und $C_{2n}$-Alkoholen gelöst, wobei die Abtrennung von $C_n$-Alkoholen aus einem Gemisch mit $C_{2n}$-Akoholen erfolgt.

Demgemäß ist Gegenstand der vorliegenden Erfindung ein Verfahren zur Herstellung von gesättigten $C_n$- und $C_{2n}$-Alkoholen mit n = 4, 5 und 6 umfassend die Verfahrensschritte

a) Aldolkondensation isomerer $C_n$-Aldehyde mit n = 4, 5 und 6 unter Erhalt eines Gemisches aus $C_n$- und $\alpha,\beta$-ungesättigten $C_{2n}$-Aldehyden, wobei der Anteil unverzweigter Aldehyde mindestens 20 Gew.-%, bezogen auf die

eingesetzten $C_n$-Aldehyde mit n = 4, 5 und 6, beträgt

b) Hydrierung des in a) erhaltenen Gemisches aus $C_n$- und α,β-ungesättigten $C_{2n}$-Aldehyden mit Wasserstoff unter Erhalt eines Gemisches aus gesättigten $C_n$ und $C_{2n}$-Alkoholen und

c) Auftrennung des Gemisches aus $C_n$ und $C_{2n}$-Alkoholen,

wobei die Auftrennung der $C_n$-Alkohole und der $C_{2n}$-Alkohole durch mindestens ein Zweikolonnensystem oder durch mindestens eine Trennwandkolonne erfolgt,
dadurch gekennzeichnet, dass das Isomerenverhältnis des erhaltenen $C_n$-Alkohols durch die in Schritt a) durchgeführte Aldolkondensation eingestellt wird, wobei der Anteil an linearen $C_n$-Alkoholen bei mindestens 10 Mol-% und weniger als 60 Mol-%, bezogen auf das Gemisch der isomeren $C_n$-Alkohole, liegt.

Die erfindungsgemäße Prozesskette erlaubt in überraschender Weise die Herstellung von gesättigten $C_n$- und $C_{2n}$-Alkoholen, die direkt für die Herstellung von Weichmachern eingesetzt werden können. Dabei hat sich insbesondere herausgestellt, dass die Isomerenverteilung, beispielsweise der $C_n$-Alkohole, dergestalt ist, dass diese nach Veresterung zu Weichmachern führen, die ein vorteilhaftes Eigenschaftsspektrum aufweisen und der Wassergehalt der $C_n$ Alkohole so gering ist, das diese ohne weitere Vorbehandlung in anhydridischen Veresterungsreaktionen oder Umesterungen eingesetzt werden können.
Wesentlich im Rahmen der vorliegenden Erfindung ist, dass das Isomerenverhältnis der erhaltenen $C_n$-Alkohole eingestellt wird. Dies erfolgt erfindungsgemäß zum einen durch die in Schritt a) durchgeführte Aldolkondensation, zum anderen durch die Steuerung bei der Auftrennung der $C_n$-Alkohole und der $C_{2n}$-Alkohole durch mindestens ein Zweikolonnensystem oder durch mindestens eine Trennwandkolonne. So lassen sich durch Selektivitätseinflüsse bei der Aldolkondensation die Isomerenverhältnisse steuern.
In der Regel zeigen die eingesetzten Aldehyde in der Aldolisierung eine unterschiedliche Reaktionsgeschwindigkeit. So wird zum Beispiel ein n-Pentanal deutlich schneller umgesetzt als ein 2-Methylbutanal.
Darüber hinaus kann das gewünschte Isomerenverhältnis auch durch die Zusammensetzung des in Schritt a) eingesetzten Stroms isomerer $C_n$-Aldehyde mit n = 4, 5 und 6 gesteuert werden. Erfindungsgemäß liegt der Anteil unverzweigter Aldehyde bei mindestens 20 Gew.-%, bezogen auf die eingesetzten $C_n$-Aldehyde mit n = 4, 5 und 6.
Die vorab beschriebenen Steuerungselemente führen dazu, dass der Anteil an linearen $C_n$-Alkoholen im erfindungsgemäß erhaltenen Alkoholgemisch bei weniger als 60 Mol-%, der Mindestgehalt an linearen $C_n$-Alkoholen im Gemisch der isomeren $C_n$-Alkohole bei mindestens 10 Mol-%, weiter bevorzugt bei mehr als 20 Mol-%, mit Vorzug dazu bei mehr als 22,5 Mol-% oder sogar bei mehr als 25 Mol-%, weiter bevorzugt bei mehr als 27,5 Mol-%, 30 Mol-% oder sogar bei mehr als 35 Mol-%.

Um die Einstellung der Isomerenverhältnisse für die $C_n$-Alkohole überwachen zu können, ist der Einsatz einer entsprechenden Analytik vorteilhaft. Im Rahmen der vorliegenden Erfindung ist insbesondere der Einsatz von gaschromatographischen Methoden bevorzugt. Hierzu eignet sich insbesondere ein Gaschromatograph des Typs 7890A der Firma Agilent mit Flammenionisationsdetektor (FID). Als Säulen werden bevorzugt Säulen des TypsHP-5 (5 % Phenyl Methyl Siloxan) mit Stickstoff als Trägergas verwendet. Aber auch geeignete spektroskopische Inline-Analytikverfahren sind hier denkbar.

Die grundsätzliche Abfolge des erfindungsgemäßen Verfahrens ist in Fig. 1 dargestellt. Im ersten Schritt werden isomere $C_n$-Aldehyde mit n = 4, 5 und 6 mittels Aldolkondensation unter Erhalt eines Gemisches aus $C_n$ und α,β-ungesättigten $C_{2n}$-Aldehyden umgesetzt.

Grundsätzlich eignen sich alle dem Fachmann bekannten $C_n$-Aldehyde mit n = 4, 5 und 6, entweder in Reinform oder in Form von Gemischen für den Einsatz in Verfahren gemäß der vorliegenden Erfindung, wobei eine Grundvoraussetzung ist, dass der Anteil unverzweigter Aldehyde mindestens 20 Gew.-%, bezogen auf die eingesetzten $C_n$-Aldehyde mit n = 4, 5 und 6, beträgt.

In das erfindungsgemäße Verfahren werden insbesondere Aldehydströme gleicher Kettenlänge eingesetzt. Der Anteil unverzweigter Aldehyde beträgt mindestens 20 Gew.-%, bezogen auf die eingesetzten $C_n$-Aldehyde mit n = 4, 5 und 6, bevorzugt mindestens 40 Gew.-%. Insbesondere beträgt der Anteil der unverzweigten Aldehyde 40 bis 95 Gew.-%, ganz besonders bevorzugt beträgt der Anteil unverzweigter Aldehyde 95 - 99,5 Gew.-%, bezogen auf die eingesetzten $C_n$-Aldehyde mit n = 4, 5 und 6,.

Vielfach werden im Rahmen von industriellen Großprozessen solche Aldehyde bzw. deren Mischungen durch Hydroformylierung aus den entsprechenden Olefinen gewonnen.

Demgemäß werden in einer bevorzugten Ausführungsform der vorliegenden Erfindung die in Schritt a) eingesetzten $C_n$-Aldehyde mit n = 4, 5 und 6 durch Hydroformylierung isomerer Olefine mit 3 bis 5 Kohlenstoffatomen mit Synthesegas in Gegenwart eines Hydroformylierungskatalysators unter Bildung der genannten Aldehyde hergestellt. Entsprechende Hydroformulierungsverfahren sind dem Fachmann bekannt und beispielsweise in Hydroformylation Fundaments, Procceses and Applications in Organic Synthesis Volume 1 & 2 Auflage 1, Franke, Börner, Willey VCH Verlag GmbH & Co, Weinheim beschrieben.

Man arbeitet in der Regel mit Rhodium- oder Kobaltkatalysatoren sowie mit oder ohne komplexstabilisierenden Zusätzen, wie organischen Phosphinen oder Phosphiten. Die Temperaturen und Drücke können, je nach Katalysator oder Olefin, in weiten Grenzen variieren. Eine Beschreibung der Hydroformylierung von Olefinen findet sich z. B. bei J. Falbe, New Syntheses with Carbon Monoxide, Springer-Verlag, Heidelberg-New York, 1980, Seite 99 ff. sowie bei Kirk-Othmer, Encyclopedia of Chemical Technology, Band 17, 4. Auflage, John Wiley & Sons, Seiten 902 bis 919 (1996).

Die Reaktionsgemische der Hydroformylierung werden vor der Aldolkondensation gemäß Schritt a) des erfindungsgemäßen Verfahrens zweckmäßigerweise zunächst vom Katalysator befreit. Wenn ein Kobaltkatalysator verwendet worden ist, kann dies durch Druckentlastung, Oxidation der im Hydroformylierungsgemisch verbliebenen Kobaltcarbonylverbindungen in Gegenwart von Wasser oder wässriger Säure und Abtrennung der wässrigen Phase geschehen. Entkobaltungsverfahren sind gut bekannt, siehe z. B. J. Falbe, a. a. O., Kirk-Othmer, a. a. O., 164, 175, BASF-Verfahren.

Wenn eine Rhodiumverbindung als Hydroformylierungskatalysator eingesetzt wird, kann man sie z. B. mittels Dünnschichtverdampfung als Destillationsrückstand abtrennen.

In der bevorzugten Ausführungsform der vorliegenden Erfindung erfolgt die Hydroformylierung gemäß dem in WO 2017/080690 beschriebenen Verfahren.
Dabei wird in der Hydroformylierung ein Katalysatorsystem eingesetzt, das Rhodium als Zentralatom enthält und mit dem Liganden (1) komplexiert ist:

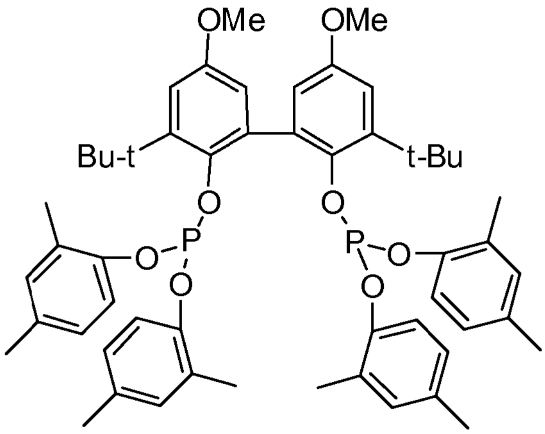

(1)

Die IUPAC Bezeichnung von Ligand (1) ist 3,3'-di-tert-butyl-5,5'-dimethoxy-[1,1'-biphenyl]-2,2'-diyltetrakis(2,4-dimethylphenyl)bis(phosphite).

Die Hydroformylierung erfolgt insbesondere bei einer Temperatur zwischen 120 °C und 140 °C. Der Druck liegt vorzugsweise zwischen $15*10^5$ Pa und $25*10^5$ Pa.
Um die Betriebsdauer zu verlängern, wird die Hydroformylierung in Gegenwart eines organischen Amins der Formel (2) durchgeführt wird,

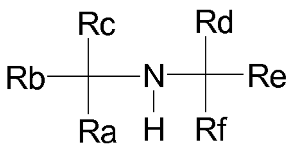

(2)

worin Ra, Rb, Rc, Rd, Re und Rf gleiche oder unterschiedliche Kohlenwasserstoffreste darstellen, die auch untereinander verbunden sein können. Das organische Amin weist vorzugsweise zumindest eine 2,2,6,6-Tetramethylpiperidineinheit auf. Konkret kann das organische Amin ein Sebacinsäuredi-4-(2,2,6,6-tetramethylpiperidinyl)-ester sein.

Es wird empfohlen, eine Rhodiumkonzentration im ersten Reaktionsgemisch zwischen 1 Gew.-ppm und 1000 Gew.-ppm einzustellen. Das Ligand/Rhodium-Verhältnis sollte zwischen 1 : 1 bis 100 : 1 betragen, wobei neben der Organophosphorverbindung gemäß Formel (1) kein weiterer Ligand als Teil des homogenen Katalysatorsystems vorzusehen ist. Im industriellen Betrieb ist nicht auszuschließen, dass aufgrund von Verunreinigungen andere Organophosphorverbindungen als 3,3'-di-tert-butyl-5,5'-dimethoxy-[1,1'-biphenyl]-2,2'-diyltetrakis(2,4-dimethylphenyl)bis(phosphite) als Teil des Katalysatorsystems an das Rhodium komplexieren. Derartige Verunreinigungen sind aber bei dem angegebenen Ligand/Rhodium-Verhältnis nicht zu berücksichtigen. Diese Angabe bezieht sich einzig auf

Ligand (1) und in beabsichtigter Weise muss kein weiterer Ligand vorgesehen sein.

Die im erfindungsgemäßen Verfahren eingesetzten isomeren $C_n$-Aldehyde mit n = 4, 5 und 6 werden erfindungsgemäß mittels Aldolkondensation, insbesondere in Gegenwart von Natronlauge, in ein Gemisch aus $C_n$- und $\alpha,\beta$-ungesättigten $C_{2n}$-Aldehyden umgewandelt. Entsprechende Verfahren sind z.B. aus DE19957522A1, DE102009045139A1, DE102009001594A1 bekannt.

Insbesondere bevorzugt erfolgt die Aldolkondensation in einem Rohrreaktor, der mindestens ein Mischmodul enthält, das den Edukt-Aldehyd in Tröpfchen mit einem durchschnittlichen Durchmesser (Sauter-Durchmesser) von 0,2 mm bis 2 mm in der kontinuierlichen Katalysatorphase (Prozesslauge) dispergiert, die aus Natronlauge und Natriumsalzen von Carbonsäuren besteht und einen Natriumgehalt von 0,6 bis 1,75 Massen-% sowie einen pH-Wert im Bereich von 12,5 bis 13,5 aufweist.

Zur Bildung der Prozesslauge wird im erfindungsgemäßen Verfahren Natronlauge verwendet. Die Natronlauge bildet zusammen mit der Rücklauge die Prozesslauge. Die Rücklauge enthält neben Natriumhydroxid Natriumsalze von Carbonsäuren, hauptsächlich von Pentansäuren. Die Carbonsäuresalze sind im Wesentlichen durch Cannizzaro-Reaktion entstanden.

Im erfindungsgemäßen Verfahren liegt der Natriumgehalt der Prozesslauge am Reaktoreingang bei 0,60 bis 1,75 Massen-%, insbesondere bei 1,1 bis 1,20 Massen-%. Zur Einstellung der Natriumkonzentration der Prozesslauge wird in die Rücklauge Natronlauge mit einer Konzentration größer als 2,5 Massen-% eingespeist. Um wenig Wasser in das Reaktionssystem einzubringen, wird bevorzugt Natronlauge mit höherer Konzentration verwendet. Im erfindungsgemäßen Verfahren wird bevorzugt Natronlauge im Konzentrationsbereich von 5 bis 30 Massen-%, beispielsweise mit 10 Massen-% verwendet.

Das erfindungsgemäße Verfahren wird in einem Rohrreaktor durchgeführt, der mindestens ein Mischmodul, bevorzugt mehrere Mischmodule aufweist. Insbesondere beträgt die Anzahl der Mischmodule 1 bis 30, ganz besonders 10 bis 20.

Ein Mischmodul versteht sich als statischer Mischer, also als ein passives Bauteil, welches keinen direkten Eigenenergiebedarf hat.

Der Rohrreaktor besteht aus einem Rohr, das bevorzugt senkrecht ausgerichtet ist. Es kann von unten nach oben oder umgekehrt durchströmt werden. Ein technischer Reaktor kann auch aus mehreren parallel angeordneten Rohren bestehen, die mit U Rohren miteinander verbunden sind.

Vorzugsweise befindet sich am Reaktoreingang ein Mischmodul. Zwischen den Mischmodulen befinden sich Leerräume. Der Volumenanteil außerhalb des/der Mischmodul(e) am Gesamtvolumen des Reaktors beträgt 20 bis 80 %, insbesondere 30 bis 60 %. Die Mischmodule können gleiche oder unterschiedliche Abstände voneinander aufweisen. Vorzugsweise nimmt der Abstand zwischen den Mischermodulen in der Fließrichtung ab. Die Abstände der Mischermodule zueinander betragen in Abhängigkeit von der vorgesehenen Leerrohrgeschwindigkeit, dem Phasenverhältnis zwischen Edukt- und Katalysatorphase, Reaktionsfortschritt und dem Mischertyp das 0,2 bis Fünffache der Mischmodullänge, insbesondere das 0,5 bis zweifache der Mischmodullänge.

Das Mischmodul besteht aus einem statischen Mischer oder aus einer Anordnung von mehreren, bevorzugt zwei statischen Mischern.

Besteht das Mischermodul aus zwei gleichen statischen Mischern sind diese bevorzugt verdreht um die Längsachse des Reaktors angeordnet, insbesondere verdreht um einen Winkel von 45° bis zu 90°. Vorzugsweise werden Mischelemente in dem Mischermodul mit einem Abstand von zwei Rohrdurchmessern angeordnet.

Ein Mischmodul kann auch aus statischen Mischern unterschiedlicher Bauart bestehen. Es kann vorteilhaft sein, dass bei einem Mischermodul, bestehend aus zwei statischen Mischern, der erste einen geringeren hydraulischen Durchmesser aufweist als der zweite. Dabei werden im ersten statischen Mischer möglichst kleine Tröpfchen erzeugt und im zweiten statischen Mischer durch Wahl eines größeren hydraulischen Durchmessers die Koaleszenz des Tropfenschwarms verhindert.

Der hydraulische Durchmesser der Mischelemente der Mischermodule nimmt mit der Fließrichtung vorzugsweise ab.

Im Reaktor können die Mischermodule gleich oder unterschiedlich sein, d. h. sie können derselben oder unterschiedlicher Bauart sein.

Als Mischelemente können alle statischen Mischer verwendet werden, die unter den vorgesehenen Reaktionsbedingungen in der Lage sind, die organische Phase in der Katalysatorphase in Tröpfchen mit einem durchschnittlichen Sauter-Durchmesser im Bereich von 0,2 bis 2,0 mm zu dispergieren.

Als statische Mischer können im erfindungsgemäßen Verfahren Mischelemente eingesetzt werden, die für die Dispergierung von zwei nicht mischbaren, niederviskosen Flüssigkeiten geeignet sind, wie sie kommerziell erhältlich sind. Erfindungsgemäß wird die Aldolkondensation der $C_n$-Aldehyde im Temperaturbereich von 100 bis 150 °C, insbesondere im Bereich von 110 bis 140 °C, ganz besonders im Bereich von 120 bis 140 °C durchgeführt.

Die Umsetzung kann in den genannten Temperaturbereichen isotherm, adiabatisch oder polytrop durchgeführt werden. Beispielsweise kann am Reaktoreingang eine Temperatur von 120 °C und am Reaktorausgang eine Temperatur

von 140 °C vorliegen.

Der Reaktionsdruck im Reaktor liegt mindestens so hoch, dass sowohl die Prozesslauge als auch die organischen Stoffe (Edukt und Produkte) jeweils als flüssige Phase vorliegen. Der Druck liegt im Bereich von 0,2 bis 1,0 MPa, vorzugsweise im Bereich 0,3 bis 0,5 MPa.

Im erfindungsgemäßen Verfahren beträgt das Mengenverhältnis [kg/kg] von Prozesslauge zu Edukt am Reaktoreingang im Bereich von 5 bis 40, insbesondere im Bereich von 10 bis 15.

Die durchschnittliche Leerrohrgeschwindigkeit des Gemisches aus Edukt und Prozesslauge (unter Annahme gleicher Strömungsgeschwindigkeit beider Phasen) in dem technischen Reaktor liegt im Bereich von 0,5 bis 4 m/s, insbesondere im Bereich von 1 bis 2,5 m/s.

Die durchschnittliche Verweilzeit des Reaktionsgemisches im Reaktor beträgt 40 bis 360 s, insbesondere 60 bis 180 s.

Im erfindungsgemäßen Verfahren weisen die in der Prozesslauge dispergierten Tröpfchen der organischen Phase nach Verlassen eines Mischermoduls einen durchschnittlichen Sauterdurchmesser von 0,2 bis 2 mm, insbesondere einen von 0,6 bis 1,3 mm auf.

Der Belastungsfaktor liegt im Bereich von 0,2 bis 0,8.

Die erhaltenen Aldehyde können optional vor der Hydrierung gemäß Schritt c) aufgearbeitet werden.

Eine Möglichkeit dazu ist es den Reaktionsaustrag zu kühlen und die organische Phase von der Laugenphase zu trennen. Vorzugsweise erfolgt die Phasentrennung im Temperaturbereich von 60 bis 130 °C, insbesondere im Bereich von 70 bis 120 °C, ganz besonders im Bereich von 90 bis 110 °C. Die Trennzeiten betragen je nach der gewählten Temperatur 3 bis 10 Minuten. Bei Temperaturen oberhalb von 90 °C liegt die Trennzeit unter 8 Minuten. Als Trennzeit wird die Zeit definiert, nach der die organische Wertproduktphase klar und frei von Spuren an heterogenem Wasser ist.

Zur Abtrennung der schweren, wässrigen Phase von der leichten, organischen Phase können Abscheider eingesetzt werden, die die Phasentrennung mit alleiniger Ausnutzung der Schwerkraft ermöglichen. Diese sogenannten Schwerkraftabscheider können auch mit Einbauten als koaleszenzfördernde Maßnahme zur Erhöhung der Trennleistung ausgeführt werden. Durch die Verwendung von Einbauten wird der Koaleszenz- und Sedimentationsprozess beschleunigt. Als Koaleszenzhilfen können z. B. Platten, Füllkörper, Gewebepackungen oder Faserbettabscheider eingesetzt werden. Schwerkraftabscheider können als liegende Behälter oder als stehende Behälter ausgeführt werden.

Alternativ zu Schwerkraftabscheidern können zur Flüssig-Flüssig-Trennung auch Separatoren nach dem Prinzip der Zentrifugen eingesetzt werden. Durch Fliehkräfte in einer sich drehenden Trommel wird dabei die schwere Phase getrennt.

Um die schwere, wässrige Phase abzutrennen, werden im erfindungsgemäßen Verfahren vorzugsweise Schwerkraftabscheider eingesetzt, bevorzugt Schwerkraftabscheider, ausgeführt als liegende Behälter mit Einbauten.

Ein Teil der abgetrennten Laugenphase wird zur Abtrennung des Reaktionswassers ausgeschleust, der andere Teil wird in den Reaktor zurückgeführt. Mit dem Ausschleusestrom werden auch ein Teil der als Nebenprodukte gebildeten Carbonsäuren (als Natriumsalze) und Natriumhydroxid abgetrennt. Dieser Strom kann einer Kläranlage zugeführt werden. Es ist jedoch auch möglich diesen Strom aufzuarbeiten und teilweise in den Prozess zurückzuführen, wie beispielsweise in DE 198 49 922 und DE 198 49 924 beschrieben.

Enthält die organische Phase neben den $C_n$- und $\alpha,\beta$-ungesättigten $C_{2n}$-Aldehyden andere Nebenprodukte, wie Carbonsäuresalze, Natriumhydroxid und gelöstes Wasser, so können Basenspuren und ein Teil der Carbonsäuresalze durch eine Wasserwäsche entfernt werden. Der dabei anfallende Wasserextrakt kann zum Ansetzen der Frischlauge verwendet werden.

Wie bereits vorab ausgeführt, erfolgt die Steuerung der Isomerenverhältnisse der erfindungsgemäß hergestellten Alkohole und anderem durch die Aldolkondensation. Insbesondere kann erfindungsgemäß der Umsatzgrad der eingesetzten Aldehyde und somit die Isomerenzusammensetzung der $C_n$ Aldehyde im Ausgangsstrom der Aldolisierung durch Steuerung der Reaktionstemperatur und der Laugenkonzentration in der Aldolisierungsreaktion gesteuert werden. Die Steuerung der Verweilzeit der eingesetzten Aldehyde ist ebenfalls ein weiteres probates Mittel, um den Umsatzgrad dieser Aldehyde zu steuern. Dies kann beispielsweise durch Veränderung des Reaktionsraumes und/oder durch Steuerung der Zulaufmenge erreicht werden. Alternativ oder im Zusammenspiel mit den vorher genannten Einflussgrößen, kann auch durch Zugabe von geeigneten Lösemitteln zum Zulaufstrom die Verweilzeit und auch die Aldehydkonzentration beeinflusst werden und somit ebenfalls der Umsatzgrad gesteuert werden.

Die so erhaltenen $C_n$- und $\alpha,\beta$-ungesättigten $C_{2n}$-Aldehyde werden gemäß Schritt b) des erfindungsgemäßen Verfahrens mit Wasserstoff unter Erhalt eines Gemisches aus gesättigten $C_n$ und $C_{2n}$-Alkoholen hydriert. Die Hydrierung erfolgt ebenfalls nach an sich bekannten Verfahren, beispielsweise im Temperaturbereich von 170 °C bis 200 °C bei einem Druck von $15*10^5$ Pa bis $30*10^5$ Pa an einem Trägerkatalysator, der als aktive Komponenten zumindest Nickel und Kupfer enthält, wie beispielsweise aus EP3037400 bekannt.

Vorzugsweise besteht der Hydrierkatalysator aus einem Trägermaterial das auf Titandioxid, Zirkondioxid, Aluminiumoxid, Siliziumoxid oder deren Mischoxide basiert, wobei auf diesem Trägermaterial hydrieraktive Metalle, insbesondere zumindest ein Element der Gruppe Kupfer, Kobalt, Nickel, Chrom aufgebracht sind.

Als Trägervorstufe können Aluminiumoxid, Alumosilikat, Siliziumdioxid, Titandioxid, Zirkondioxid eingesetzt werden. Eine bevorzugte Trägervorstufe ist Aluminiumoxid, insbesondere $\gamma$-Aluminiumoxid. Der Katalysator kann eines oder mehrere der hydrieraktiven Metalle enthalten. Vorzugsweise enthält der erfindungsgemäße Katalysator die Metalle Kupfer, Chrom, Nickel. Besonders bevorzugt enthält der Katalysator als hydrieraktives Metall die Kombination der drei Metalle Kupfer, Chrom und Nickel.

Der Gesamtgehalt an hydrieraktiven Metallen liegt, bezogen auf den reduzierten Katalysator, im Bereich von 1 bis 40 Massen-%, insbesondere im Bereich von 5 bis 25 Massen-%, berechnet als Metall.

Die erfindungsgemäßen Katalysatoren werden zweckmäßig in einer Form hergestellt, die bei der Hydrierung einen geringen Strömungswiderstand bietet, wie beispielsweise Tabletten, Zylinder, Strangextrudate oder Ringe. Bei der Herstellung des Katalysators wird üblicherweise das Trägervormaterial in entsprechende Form gebracht. Geformtes Trägervormaterial ist auch kommerziell erhältlich.

Im erfindungsgemäßen Verfahren kann die Hydrierung an suspendierten feinteiligen oder geformten, im Festbett angeordneten Katalysatoren kontinuierlich oder diskontinuierlich durchgeführt werden. Die kontinuierliche Hydrierung an einem im Festbett angeordneten Katalysator, bei der sich unter Reaktionsbedingungen die Produkt/Eduktphase hauptsächlich im flüssigen Zustand befindet, wird bevorzugt.

Wird die Hydrierung kontinuierlich an einem im Festbett angeordneten Katalysator durchgeführt, ist es zweckmäßig, den Katalysator vor der Hydrierung in die aktive Form zu überführen. Dies kann durch Reduktion des Katalysators mit wasserstoffhaltigen Gasen nach einem Temperaturprogramm erfolgen. Dabei kann die Reduktion gegebenenfalls in Gegenwart einer flüssigen Phase, die über den Katalysator geleitet wird, durchgeführt werden, wie etwa in DE 199 33 348 beschrieben.

Das erfindungsgemäße Verfahren wird in der Rieselphase oder bevorzugt in der Flüssigphase in Dreiphasenreaktoren im Gleichstrom durchgeführt, wobei der Wasserstoff in an sich bekannter Weise in dem flüssigen Edukt/Produkt-Strom fein verteilt wird. Im Interesse einer gleichmäßigen Flüssigkeitsverteilung, einer verbesserten Reaktionswärmeabfuhr und einer hohen Raum-ZeitAusbeute werden die Reaktoren vorzugsweise mit hohen Flüssigkeitsbelastungen von 15 bis 120, insbesondere von 25 bis 80 $m^3$ pro $m^2$ Querschnitt des leeren Reaktors und Stunde betrieben. Wird ein Reaktor isotherm und im geraden Durchgang betrieben, so kann die spezifische Katalysatorbelastung (LHSV) Werte zwischen 0,1 und 10 $h^{-1}$ annehmen.

Das erfindungsgemäße Verfahren wird mit Wasserstoff in einem Druckbereich von 5 bis 100 bar, vorzugsweise zwischen 5 und 40 bar, besonders bevorzugt im Bereich von 10 bis 25 bar durchgeführt. Die Hydriertemperaturen liegen zwischen 120 und 220 °C, insbesondere zwischen 140 und 190 °C.

Der für die Hydrierung eingesetzte Wasserstoff kann inerte Gase wie beispielsweise Methan oder Stickstoff enthalten. Vorzugsweise wird Wasserstoff mit einer Reinheit größer als 98 %, insbesondere größer als 99 % eingesetzt.

Für das erfindungsgemäße Verfahren können unterschiedliche Verfahrensvarianten gewählt werden. Es kann adiabatisch, oder praktisch isotherm, d. h. mit einem Temperaturanstieg kleiner 10 °C, ein- oder mehrstufig durchgeführt werden. Im letzteren Falle kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die Carbonylverbindungen oder Gemische von Carbonylverbindungen in Gegenwart von Wasser im geraden Durchgang oder mit Produktrückführung zu hydrieren.

Die Hydrierung setzt neben den $\alpha,\beta$-ungesättigten $C_{2n}$-Aldehyden, auch die in der Aldolisierung nicht umgesetzten $C_n$-Ausgangsaldehyde zu den entsprechenden gesättigten Alkoholen um.

An die Hydrierung schließt sich erfindungsgemäß gemäß Schritt c) die Auftrennung des Gemisches aus $C_n$ und $C_{2n}$-Alkoholen an, wobei die Auftrennung der $C_n$-Alkohole und der $C_{2n}$-Alkohole durch mindestens ein Zweikolonnensystem oder durch mindestens eine Trennwandkolonne erfolgt.

Wesentlich im Rahmen der vorliegenden Erfindung ist der Einsatz eines Zweikolonnensystems oder einer Trennwandkolonne, um in beiden Fällen die gesättigten $C_n$- und $C_{2n}$-Alkohole als Wertprodukte zu erhalten.

Die verschiedenen Verfahrensvarianten werden nachfolgend näher erläutert.

In der einen Ausführungsform der vorliegenden Erfindung kommt mindestens ein Zweikolonnensystem zum Einsatz. In der ersten Kolonne werden im Laufe des Verfahrens entstandene Alkane (in Falle von Butenen hauptsächlich Nonan) zusammen mit dem $C_n$-Alkohol (beispielsweise 2-Methylbutanol) und Wasser über Kopf genommen. Dort findet eine Phasentrennung statt und die organische Phase wird der Kolonne wieder als Rücklauf zugeführt. Durch diese Aceotroptrocknung wird der Wassergehalt im Sumpf kleiner 1000 ppm erhalten. In der zweiten Kolonne wird der $C_n$ Alkohol über Kopf genommen und damit von den Hochsiedern getrennt. Durch entsprechende Fahrweise der Kolonne kann die Isomeren-Zusammensetzung der C5 Alkohole zusätzlich gesteuert werden.

Fig.2 zeigt den detaillierten Ablauf der Auftrennung des Gemisches aus $C_n$ und $C_{2n}$-Alkoholen mittels eines Zweikolonnensystems der sich in folgende Unterschritte unterteil:

I. Destillative Auftrennung (G) des Rohproduktstromes aus der Hydrierung gemäß Schritt b) in einen Leichtsiederstrom (6) und in einen Hochsiederstrom (15). Dabei enthält der Leichtsiederstrom (6) als Hauptprodukt die Alkohole

der Einsatzaldehyde, weitere Stoffe bzw. Azeotrope die einen geringeren Siedepunkt aufweisen als die aus den $\alpha,\beta$-ungesättigten $C_{2n}$-Aldehyden entstandenen Alkohole und heterogenes Wasser. Der Hochsiederstrom (15) enthält als Hauptprodukt die aus den $\alpha,\beta$-ungesättigten $C_{2n}$-Aldehyden entstandenen Alkohole und Hochsieder bzw. Azeotrope, die einen höheren Siedepunkt aufweisen als die $C_{2n}$-Alkohole.

II. Abtrennen des heterogenen Wassers (7) aus der Destillatfraktion (6) über Abscheider (F).

III. Destillative Auftrennung (H) der organischen Phase (8) aus (F), in einen Leichtsiederstrom (9) und einen Hochsiederstrom (12). Der Leichtsiederstrom (9) enthält Stoffe bzw. Azeotrope, die einen geringeren Siedepunkt aufweisen als der höchstsiedende $C_n$-Alkohol und kann nach Kondensation heterogenes Wasser (11) enthalten. Der Hochsiederstrom enthält hauptsächlich die $C_n$-Alkohole und Stoffe bzw. Azeotrope, die einen höheren Siedepunkt aufweisen, als der höchstsiedende $C_n$-Alkohol.

IV. Abtrennen des heterogenen Wassers (11) durch Phasentrennung (E). Die organische Phase wird als Rücklauf (19) wieder auf die Kolonne (H) gefahren. Ein Teil der anfallenden organischen Phase (10) wird aus dem System gefahren.

V. Destillative Auftrennung (I) des Hochsiederstroms (12) in einen Leichtsiederstrom (4) und in einen Hochsiederstrom (14). Der Leichtsiederstrom (4) enthält hauptsächlich den $C_n$-Alkohol mit der gewünschten Isomerenverteilung. Der Hochsiederstrom (14) enthält hauptsächlich Stoffe bzw. Azeotrope, die einen höheren Siedepunkt aufweisen, als der höchstsiedende $C_n$-Alkohol.

VI. Destillative Auftrennung des Hochsiederstroms (15) in einen Leichtsiederstrom (5), der die $C_{2n}$-Alkohole enthält und in einen Hochsiederstrom (18). Der Leichtsiederstrom (5) enthält hauptsächlich den $C_{2n}$-Alkohol. Der Hochsiederstrom (18) enthält hauptsächlich Stoffe bzw. Azeotrope, die einen höheren Siedepunkt aufweisen, als der höchstsiedende $C_{2n}$-Alkohol.

Die in den Kolonnen eingesetzten Temperaturprofile werden in Abhängigkeit von der jeweiligen Zusammensetzung des Produktstroms aus der Hydrierung (Schritt b) des erfindungsgemäßen Verfahrens) jeweils angepasst. Beispielsweise kann der Produktstrom (3) folgende Zusammensetzung aufweisen:

| | |
|---|---|
| 2-Propylheptenal (Masse %) | 0,9 |
| n-Pentanol (Masse %) | 2,8 |
| 2-Methylbutanol (Masse %) | 2,8 |
| 2-Propylheptanol (Masse %) | 87,5 |
| 2-Propyl-4-Methylhexanol (Masse %) | 4,2 |
| $H_2O$ (Masse %) | 1,7 |

Bei dieser Zusammensetzung wird vorzugsweise bei einem Kopfdruck von 0.2 bar(abs) und einem Druckverlust von 0.1 bar in der Kolonne (G) gearbeitet, wobei eine Sumpftemperatur von 176 °C und eine Kopftemperatur von 65 °C, oder entsprechende Äquivalente, einzuhalten sind. In Kolonne (H) wird insbesondere bei einem Kopfdruck von 1.05 bar(abs) und einem Druckverlust von 0.03 bar bei einer Sumpftemperatur von 137 °C und eine Kopftemperatur von 94 °C, oder entsprechende Äquivalenten, gearbeitet. In der Kolonne (I) sind in der Regel bei einem Kopfdruck von 1.08 bar(abs) und einer Druckdifferenz von 0.03 bar eine Sumpftemperatur von 178 °C und eine Kopftemperatur von 134 °C, oder entsprechende Äquivalente, einzuhalten.

Die Kolonne (J) wird bei einem Druck von 0.15 bar(abs) und einer Druckdifferenz von 0.006 bar einer Sumpftemperatur von 175 °C und einer Kopftemperatur 158 °C, oder entsprechenden Äquivalenten, gefahren.

Die genannten Temperaturprofile sind als beispielhaft anzusehen. Weitere Ausgestaltungen, insbesondere im Zusammenhang mit variierenden Feedzusammensetzungen sind im Rahmen der vorliegenden Erfindung mit umfasst,

In einer weiteren, ebenso bevorzugten Ausführungsform der vorliegenden Erfindung kommt mindestens eine Trennwandkolonne zum Einsatz. In einer Trennwandkolonne können die in für das Zweikolonnensystem beschrieben Trennoperationen in einer Kolonne zusammengefast werden. Über Kopf wird ebenfalls das beispielsweise Alkan/2-Methylbutanol/Wassergemisch erhalten, analog einer Phasentrennung unterzogen und die organische Phase der Kolonne wieder als Rücklauf zugeführt. Über einen Seitenabzug im Bereich der Trennwand wird der spezifikationsgerechte $C_n$-Alkohol abgezogen. Das Isomerenverhältnis des $C_n$-Alkohols im Seitenabzug kann über die Destillatabnahme der organischen Phase und Anteil des Alkohols im Sumpf der Kolonne in gewissen Grenzen gesteuert werden. In Fig. 3 ist der Ablauf bei Einsatz einer Trennwandkolonne (K) für die Auftrennung des Leichtsiederstroms (6) dargestellt.

Hierbei werden die in Fig. 2 dargestellten Kolonnen H und I zu einer Trennwandkolonne (K) zusammengefasst (siehe Fig. 3). Bei der Trennwandkolonne (K) sind bei einem Kopfdruck von 1.05 bar(abs) und einem Druckverlust von 0.06 bar eine Sumpftemperatur von 180 °C, eine Kopftemperatur von 118 °C und eine Produkttemperatur von 134 °C,

oder entsprechende Äquivalente, einzuhalten.

Fig. 4 zeigt eine weitere bevorzugte Ausführungsform, bei der auch die Auftrennung des Rohproduktstromes aus der Hydrierung b) über eine Trennwandkolonne realisiert wird. In diesem Falle wird über einen Seitenabzug im Bereich der Trennwand der $C_{2n}$-Alkohol abgezogen.

Das erfindungsgemäße Verfahren eignet sich in vorteilhafter Weise für die Herstellung von $C_n$- und $C_{2n}$-Alkoholen aus Aldehyden bzw. im Falle der vorgelagerten Hydroformylierung aus Olefinen.

Bei den eingesetzten isomeren $C_n$-Aldehyden mit n = 4, 5 und 6 ist n = 5 insbesondere bevorzugt. Wie bereits vorab ausgeführt, können solche Aldehyde durch Hydroformylierung entsprechender isomerer Olefine erhalten werden. Bei letzteren handelt es sich insbesondere um Olefine mit 3 bis 5 Kohlenstoffatomen, namentlich Propene, Butene und Pentene, wobei die jeweiligen Stellungsisomere (bei Butenen und Pentenen) durch die genannten Bezeichnungen mit umfasst sind. Insbesondere bevorzugt handelt es sich bei den isomeren Olefinen um Butene. Letztere werden mittels des erfindungsgemäßen Verfahrens zu Pentanolgemischen und Decanolgemischen umgesetzt.

Im Hinblick auf die Weichmachersynthesen sind insbesondere die Isomerenverteilungen der Pentanolgemische von besonderem Interesse,

Beispielhaft und als bevorzugte Ausführungsform enthält das erhaltene Pentanolgemisch vorzugsweise weniger als 60 Mol-% n-Pentanol. Der Mindestgehalt an n-Pentanol im Gemisch der isomeren Pentanol liegt vorzugsweise bei mindestens 2 Mol-%, bevorzugt bei mindestens 10 Mol-%, weiter bevorzugt bei mehr als 20 Mol-%, mit Vorzug dazu bei mehr als 22,5 Mol-% oder sogar bei mehr als 25 Mol-%, weiter bevorzugt bei mehr als 27,5 Mol-%, 30 Mol-% oder sogar bei mehr als 35 Mol-%. Neben den linearen n-Pentylresten enthalten die besonders bevorzugten Pentanole verzweigte Pentylreste. Ein verzweigter Pentylrest ist vorzugsweise ein Methylbutylrest. Dementsprechend ist ein Pentanolgemisch bevorzugt, in welchen die verzweigten Pentylreste zu mindestens 50 Mol-%, bevorzugt zu mindestens 60 Mol-%, weiter bevorzugt zu mindestens 70 Mol-%, bevorzugt dazu zu mindestens 80 Mol-%, ganz besonders bevorzugt zu mindestens 90 Mol-% und insbesondere zu mindestens 95 Mol % aus Methylbutylresten bestehen.

Vorteilhaft ist, wenn die verzweigten isomeren Pentylreste einen großen Anteil an 2 Methylbutylresten aufweisen. In einer bevorzugten Ausführungsform sind deshalb mindestens 50 Mol-%, vorzugsweise mindestens 60 Mol-%, bevorzugt mindestens 70 Mol-%, weiter bevorzugt mindestens 80 Mol-%, besonders bevorzugt mindestens 90 Mol-% und insbesondere mindestens 95 Mol-% der verzweigten isomeren Pentylreste 2-Methylbutylreste. Die bevorzugten Pentanolgemische enthalten vorzugsweise 20 bis 95 Mol-%, bevorzugt 30 bis 85 Mol % und insbesondere 40 bis 75 Mol-% 2-Methylbutylreste, bezogen auf alle enthaltenen Pentylreste.

In einer besonders bevorzugten Ausführungsform besteht das Pentanolgemisch zu mindestens 75 Mol-%, weiter bevorzugt zu mindestens 90 Mol-% und insbesondere zu mindestens 95 Mol-% aus Pentanolen, welche - vorzugsweise ausschließlich - 2 Methylbutyl- und/oder lineare Pentylreste enthalten, wobei das Molverhältnis von 2 Methylbutylresten zu linearen Pentylresten innerhalb dieses Pentanolgemisches vorzugsweise im Bereich von 99 : 1 bis 40 : 60, insbesondere im Bereich von 70 : 30 bis 40 : 60 liegt.

Die Einstellung der gewünschten Produkteigenschaften, insbesondere der vorab genannten Isomerenverteilungen, erfolgt insbesondere durch die bei den jeweiligen Verfahrensschritten a), b) und c) beschriebenen Parameter, insbesondere durch die bei der Aldolisierung eingesetzte Natronlaugekonzentration und die dort genannten Temperaturen (siehe Schritt a)) sowie durch die Parameter zur Durchführung der in Schritt c) genannten Auftrennung des Gemisches aus $C_n$ und $C_{2n}$-Alkoholen durch die erfindungsgemäßen Zweikolonnen- oder Trennwandkolonnensysteme.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keinesfalls als in irgendeiner Weise limitierende Offenbarung aufzufassen.

Nachfolgend wird die vorliegende Erfindung anhand von Beispielen näher erläutert. Alternative Ausführungsformen der vorliegenden Erfindung sind in analoger Weise erhältlich.

**Beispiele:**

Für die Durchführung der Beispiele und die Ermittlung der jeweiligen Parameter wurden folgende grundsätzlichen Annahmen verwendet, bezogen auf das System $C_n/C_{2n}$ mit n = 5.

Die Berechnung des Umsatzgrades Xi1 und Xi2 erfolgte nach folgender Berechnungsvorschrift:

$$\mathrm{Xi1} = \frac{\text{Masse\% nPentanal Edukt Eingang} - \text{Masse\% nPentanal Produkt Ausgang}}{\text{Masse\% nPentanal Edukt Eingang}}$$

$$\mathrm{Xi2} = \frac{\text{Masse\% 2Methylbutanal Edukt Eingang} - \text{Masse\% 2Methylbutanal Produkt Ausgang}}{\text{Masse\% 2Methylbutanal Edukt Eingang}}$$

Die Berechnung des n-Pentanalanteils bezogen auf die C5-Aldehyde im Produkt (Sn1) erfolgte nach folgender Berechnungsvorschrift:

$$Sn1 = \frac{\text{Masse\% nPentanal Edukt Ausgang}}{\text{Masse\% nPentanal Produkt Ausgang} + \text{Masse\% 2Methylbutanal Produkt Ausgang}}$$

Die Berechnung des 2-Propylheptenalanteils bezogen auf die ungesättigten C10-Aldehyde im Produkt (Sn2) erfolgte nach folgender Berechnungsvorschrift:

$$\text{Sn2} = \frac{\text{Masse\% 2Propylheptenal}}{\text{Masse\% 2Propylheptenal} + \text{Masse\% 4Propyl4Methylhexenal}}$$

Die stofflichen Zusammensetzungen in den Beispielen wurden mittels Gaschromatographie ermittelt. Hierzu wurde ein Gaschromatograph 7890A der Firma Agilent mit Flammenionisationsdetektor (FID) eingesetzt. Als Säule wurde eine HP-5 (5% Phenyl Methyl Siloxan) 325 °C 30m × 320μm × 0.25μm verwendet.
Folgendes Temperaturprogramm wurde für die Analyse angewendet: 2 min bei 50 °C, dann auf 150 °C mit 10 °C/min anschließend mit 40 °C/min auf 250 °C mit 10 min Haltephase. Injektionsvolumen 0.5μl. Injektortemperatur 250 °C Trägergas Stickstoff mit einem Fluss von 105 ml/min und einem Split von 50 : 1

**Beispiel 1:** Aldolisierung von n-Pentanal inklusive Kondensation zu α,β-ungesättigten C10 Aldehyden in einem Rührreaktor:

Die α,β-ungesättigten C10 Aldehyde wurden in einem Rührreaktor hergestellt. Dieser Reaktor hatte die Form einer Extraktionskolonne mit einem Volumen von 2.1 Liter. Reaktor war in zehn Mischkammern unterteilt und jede dieser Mischkammern war mit einem 4 Blatt-Rührer ausgestattet, die auf einer gemeinsamen Rührwelle angebracht waren. Eine Katalysatorphase wurde durch eine Kreislaufpumpe über den Reaktor im Kreis geführt. Als Katalysatorphase kam eine 2%ige wässrige Natronlaugelösung zum Einsatz. Der Katalysatorkreislauf betrug über alle Versuche 80 l/h. Das Edukt enthielt 98,3 Masse-% n-Pentanal, die zu 100% fehlenden Anteile setzten sich aus Nebenkomponenten zusammen. Das Edukt wurde dem Katalysatorkreislauf kurz vor dem Reaktoreingang kontinuierlich mit 8l/h zugeführt.
Das am Reaktorausgang anfallende zwei Phasen Gemisch wurde in einem Phasentrennbehälter in eine organische Produktphase und eine Katalysatorphase getrennt.
Die Anlage wurde unter Stickstoffatmosphäre bei einem Druck von 4bar betrieben und nach drei Stunden stationärem Betrieb, wurden folgende Ergebnisse ermittelt.

| Reaktionsbedingungen | | | |
|---|---|---|---|
| Rührerdrehzahl u/min | 500 | 1000 | 2000 |
| Temperatur °C | 130 | 130 | 130 |
| Produkt-Zusammensetzung | | | |
| n-Pentanal (Masse %) | 9,7 | 4,9 | 4,9 |
| 2-Propylheptenal (Masse %) | 85,4 | 91,2 | 91,3 |
| Rest (incl. gelöstes Wasser) | 4,9 | 3,9 | 3,8 |
| Umsatzgrad | | | |
| n-Pentanal | 0,91 | 0,95 | 0,95 |
| | | | |
| Ausgang organische Phase kg/h | 5.9 | 5.8 | 5.8 |
| Massenstrom Pentanal kg/h | 0.6 | 0.3 | 0.3 |
| Massenstrom 2-Propylheptenal kg/h | 5.0 | 5.8 | 5.8 |

**Beispiel 2:** Aldolisierung von n-Pentanal und 2-Methylbutanal inklusive Kondensation zu α,β-ungesättigten C10 Aldehyden in einem Strömungsrohr:

Die α,β-ungesättigten C10 Aldehyde wurden in einem drei Meter langen DN15 Rohr, mit einem Innendurchmesser von 17,3mm, hergestellt. Dieses Rohr hatte das Volumen von 6,8 Liter. Circa 50 % des Gesamtvolumens war mit statischen Mischern mit einem Kanaldurchmesser von 2 mm gefüllt. Die Mischelemente sind aus geriffelten Lamellen aufgebaut, die offene, sich kreuzende Kanäle bilden. Die Kanäle sind mit einem Abstand von einer Mischlänge um 90° zueinander versetzt im Rohr angeordnet. Über dieses Rohr wurde mit Hilfe einer Pumpe ein kontinuierlicher Kreislauf einer Katalysatorphase von 80l/h aufgebaut. Als Katalysatorphase kam eine 2,1%ige wässrige Natronlauge Lösung zum Einsatz.

Das Edukt enthielt 94,4 Masse-% n-Pentanal und 5,0 Masse % 2-Methylbutanal, die zu 100 % fehlenden Anteile setzten sich aus Nebenkomponenten zusammen. Das Edukt wurde dem Katalysatorkreislauf kurz vor Beginn der statischen Mischer kontinuierlich mit 8 l/h zugeführt.

Das am Reaktorausgang anfallende zwei Phasen Gemisch wurde in einem Phasentrennbehälter in eine organische Produktphase und eine Katalysatorphase getrennt.

Die Anlage wurde unter Stickstoffatmosphäre bei einem Druck von 4 bar betrieben und nach drei Stunden stationärem Betrieb, wurden folgende Ergebnisse ermittelt.

| Reaktionsbedingungen | | | |
|---|---|---|---|
| Temperatur °C | 110 | 120 | 130 |
| Produkt-Zusammensetzung | | | |
| n-Pentanal (Masse %) | 19.0 | 11.3 | 2.8 |
| 2-Methylbutanal (Masse %) | 3.5 | 2.5 | 2.8 |
| 2-Propylheptenal (Masse %) | 72.5 | 80.5 | 85.4 |
| 4-Propyl-4-Methylhexenal (Masse %) | 3.6 | 3.6 | 4.0 |
| Rest (incl. gelöstes Wasser) | 1.4 | 2.1 | 5.0 |
| Umsatzgrad n-Pentanal (Xi1) | 0.81 | 0.89 | 0.97 |
| Umsatzgrad 2-Methylbutanal(Xi2) | 0.36 | 0.53 | 0.5 |
| n-Pentanalanteil bezogen auf die C5-Aldehyde im Produkt (Sn1) | 84.6% | 81.7% | 50.0% |
| 2-Propylheptenalanteil bezogen auf die ungesättigten C10-Aldehyde im Produkt (Sn2) | 95.3% | 95.7% | 95.5% |
| Ausgang organische Phase incl. gelöstem Wasser kg/h | 1.5 | 1.5 | 1.5 |
| Massenstrom C5-Aldehyde im Ausgang kg/h | 0.3 | 0.2 | 0.08 |
| Massenstrom α,β-ungesättigten C10-Aldehyde im Ausgang kg/h | 1.1 | 1.2 | 1.3 |

**Beispiel 3:** Aldolisierung von n-Pentanal und 2-Methylbutanal inklusive Kondensation zu α,β-ungesättigten C10 Aldehyden in einem Strömungsrohr:

Die α,β-ungesättigten C10 Aldehyde wurden in einem vier Meter langen DN15 Rohr, mit einem Innendurchmesser von 17,3 mm, hergestellt. Dieses Rohr hatte das Volumen von 9,1 Liter. Circa 50 % des Gesamtvolumens war mit statischen Mischern mit einem Kanaldurchmesser von 2mm gefüllt. Die Mischelemente sind aus geriffelten Lamellen aufgebaut, die offene, sich kreuzende Kanäle bilden. Die Kanäle sind mit einem Abstand von einer Mischlänge um 90° zueinander versetzt im Rohr angeordnet. Über dieses Rohr wurde mit Hilfe einer Pumpe ein kontinuierlicher Kreislauf einer Katalysatorphase von 80 l/h aufgebaut. Als Katalysatorphase kam eine 2,1%ige wässrige Natronlauge Lösung zum Einsatz.

Das Edukt enthielt 93,7 Masse-% n-Pentanal und 5,2 Masse % 2-Methylbutanal, die zu 100 % fehlenden Anteile setzten sich aus Nebenkomponenten zusammen. Das Edukt wurde dem Katalysatorkreislauf kurz vor Beginn der statischen Mischer kontinuierlich zugeführt. In diesem Beispiel wurde die Eduktmenge variiert.

Das am Reaktorausgang anfallende zwei Phasen Gemisch wurde in einem Phasentrennbehälter in eine organische Produktphase und eine Katalysatorphase getrennt.

Die Anlage wurde unter Stickstoffatmosphäre bei einem Druck von 4bar betrieben und nach vier Stunden stationärem Betrieb, wurden folgende Ergebnisse ermittelt.

| | | | |
|---|---|---|---|
| Reaktionsbedingungen | | | |
| Temperatur °C | 130 | 130 | 130 |
| Edukt l/h | 2.2 | 4.1 | 8.4 |
| Produkt-Zusammensetzung | | | |
| n-Pentanal (Masse %) | 3.7 | 5.0 | 11.0 |
| 2-Methylbutanal (Masse %) | 2.6 | 2.3 | 2.3 |
| 2-Propylheptenal (Masse %) | 87.6 | 86.7 | 80.4 |
| 4-Propyl-4-Methylhexenal (Masse %) | 4.6 | 3.8 | 3.5 |
| Rest | 1.5 | 2.2 | 2.8 |
| Umsatzgrad | | | |
| n-Pentanal(Xi1) | 0.96 | 0.95 | 0.89 |
| 2-Methylbutanal(Xi2) | 0.54 | 0.6 | 0.6 |
| n-Pentanalanteil bezogen auf die C5-Aldehyde im Produkt (Sn1) | 58,2% | 68,7% | 82,8% |
| 2-Propylheptenalanteil bezogen auf die ungesättigten C10-Aldehyde im Produkt (Sn2) | 95,1% | 95,8% | 95,8% |
| Ausgang organische Phase incl. gelöstem Wasser kg/h | 1.6 | 3.0 | 6.1 |
| Massenstrom C5-Aldehyde im Ausgang kg/h | 0.1 | 0.2 | 0.8 |
| Massenstrom $\alpha$,$\beta$-ungesättigten C10-Aldehyde im Ausgang kg/h | 1.5 | 2.7 | 5.1 |

**Beispiel 4**

Die in Beispiel 2 bei 110 °C und 130 °C entstandenen Produktströme wurden einer Hydrierung unterzogen. Zur Hydrierung wurde eine Kreislaufanlage analog zu DE102009045718A1 Beispiel 1 verwendet. Analog dazu wurden 105 g Katalysator mit Cu (6 Masse %)/Ni (3,1 Masse %)/Cr (0,6 Masse %) auf $Al_2O_3$ als Strangextrudate mit 1,5 mm Durchmesser und einer Länge von 3 - 5 mm eingesetzt. Die Reaktionsbedingungen betrugen 180 °C und 25 bar absolut. Der Eduktzulauf betrug 100ml/h bei einer Kreislaufmenge von 40 l/h und einer Abgasmenge von 60 Nl/h.

| Feed | 110°C aus Beispiel 2 | 130°C aus Beispiel 2 |
|---|---|---|
| Produkt-Zusammensetzung | | |
| n-Pentanal (Masse %) | 0,2 | 0,0* |
| 2-Methylbutanal (Masse %) | 0,0* | 0,0* |
| 2-Propylheptenal (Masse %) | 0,7 | 0,9 |
| 4-Propyl-4-Methylhexanal (Masse %) | 0,0* | 0,0* |
| n-Pentanol (Masse %) | 18,7 | 2,7 |
| 2-Methylbutanol (Masse %) | 3,4 | 2,7 |
| 2-Propylheptanol (Masse %) | 71,0 | 83,7 |
| 4-Propyl-4-Methylhexanol (Masse %) | 3,5 | 4,0 |
| Rest | 2,5 | 6,0 |
| n-Pentanolanteil bezogen auf die C5-Alkohole im Produkt | 84,4% | 50,0% |
| 2-Propylheptanolanteil bezogen auf die C10-Alkohole im Produkt | 95,3% | 95,4% |
| *unter Nachweisgrenze | | |

**Beispiel 5**

Für die Betrachtung der Aufreinigung des Produktstroms aus der Hydrierung wurden Simulationsstudien durchgeführt. Die in Beispiel 4 entstandenen Produktströme werden in die einzelnen Fraktionen, nach Abbildung 2 aufgetrennt. Verwendet wurde dazu Aspen Plus V7.3 mit der Methode PSRK (predited Soave-Redlich-Kwong). Die Kolonnen werden als Radfrac-Modelle gerechnet und die Kolonnen so ausgelegt, dass eine Abtrennung der Produkte mit einer Reinheit von ca. 99,9 % möglich ist. Zur Erreichung der Produktqualitäten sind vier Kolonnen mit unterschiedlichen Spezifikationen vorgesehen.

| | Theoretische Trennstufen |
|---|---|
| Kolonne I | 65 |
| Kolonne II | 20 |
| Kolonne III | 10 |
| Kolonne IV | 55 |

Durch die Zugabe von Wasser (im Feed der Kolonne I) wird die Trennung der Leichtsieder begünstigt. Damit sich das Wasser nicht in den Kolonnen anreichert und eine wässrige Phase entsteht, wird Wasser am Kopf jeder Kolonne in einem Dekanter abgetrennt.

| | Feed | Produktstrom 1 (Alkohol aus $\alpha$,$\beta$-Aldehyden) | Produktstrom 2 (Alkohol aus Einsatzaldehyden) |
|---|---|---|---|
| 2-Propylheptenal (Masse %) | 0,9 | 0 | 0 |
| n-Pentanol (Masse %) | 2,8 | 0 | 55,5 |
| 2-Methylbutanol (Masse %) | 2,8 | 0 | 44,4 |
| 2-Propylheptanol (Masse %) | 87,5 | 95,4 | 0 |
| 2-Propyl-4-Methylhexanol (Masse %) | 4,2 | 4,6 | 0 |
| $H_2O$ | 1,7 | 0 | 0 |

**Beispiel 6 (Vergleich)**

Alternativ zur Abtrennung mit 4 Kolonnen kann die Trennung mit Hilfe von Trennwandkolonnen durchgeführt werden (ebenfalls mit Aspen Plus V7.3, PSRK und Radfrac simuliert). Werden z.B. die Kolonne 2 und 3 durch eine 31 stufige Trennwandkolonne ersetzt, kann der Alkohol der Einsatzaldehyde als mittlere Fraktion abgezogen werden.
Die Trennwand erstreckt sich dabei über 14 der 31 Trennstufen. Das Produkt wird auf der 15. Stufe abgeführt. Der von unten auf die Trennwand treffende Dampfstrom wird zu gleichen Teilen auf die beiden Kolonnensegmente aufgegeben, wohingegen 75 % des Flüssigkeitsstroms auf die Produktseite und 25 % auf die Feedseite geleitet wird.
Wird das Kopfprodukt der Kolonne 1 auf die Trennwandkolonne geleitet ergibt sich folgende Produktverteilung:

| | Feed zur Trennwandkolonne | Produkt (Seitenstrom der Trennwandkolonne) |
|---|---|---|
| $H_2O$ (Masse %) | 7,4 | 0 |
| n-Pentanol (Masse %) | 39,3 | 66,9 |
| 2-Methylbutanol (Masse %) | 39,5 | 33,1 |
| 2-Propylheptanol (Masse %) | 0 | 0 |

(fortgesetzt)

| | Feed zur Trennwandkolonne | Produkt (Seitenstrom der Trennwandkolonne) |
|---|---|---|
| 2-Propyl-4-Methylhexanol (Masse %) | 0,6 | 0 |
| 2-Propylheptenal (Masse %) | 13,2 | 0 |

Die Ergebnisse zeigen das durch Variation der Isomeren-Zusammensetzung der eingesetzten Aldehyde, durch Beeinflussung des Stoffüberganges, durch Temperatur und Verweilzeit des Eduktes in dem Aldolisierungsreaktionsschritt, mit anschließender Hydrierung und gezielter Destillation der Alkohole aus den Einsatzaldehyden und dem Alkohol aus $\alpha,\beta$-ungesättigten Aldehyden, die Mengen und Isomeren-Verteilung der Alkohole gezielt gesteuert werden kann.

**Bezugszeichenliste**

1 Hydroformylierungsprodukt
2 Aldolisierungsprodukt
3 Hydrierungsprodukt (beispielsweise mit n= 5 im Wesentlichen Wasser, Butan, Buten, $C_5$-Aldehyde, 2-Metylbutanol, Pentanol, Nonan, $\alpha,\beta$-ungesättigte $C_{10}$ Aldehyde, $C_{10}$-Aldehyde, $\alpha,\beta$-ungesättigte $C_{10}$-Alkohole, $C_{10}$-Alkhohole, C10+-Oligomere mit mehr als 10 Kohlenstoffatomen)
4 $C_n$-Alkohol (beispielsweise mit n= 5 im Wesentlichen 2-Metylbutanol, Pentanol)
5 $C_{2n}$-Alkohol (beispielsweise mit n= 5 im Wesentlichen $C_{10}$-Alkhohole)
6 Leichtstrom $C_{10}$-Alkohol abgereichert mit Anteilen heterogenem Wasser (beispielsweise mit n= 5 im Wesentlichen Wasser (heterogen), Butan, Buten, $C_5$-Aldehyde, 2-Metylbutanol, Pentanol, Nonan, $\alpha,\beta$-ungesättigte $C_{10}$ Aldehyde, $C_{10}$-Aldehyde, $\alpha,\beta$-ungesättigte $C_{10}$-Alkohole)
7 wässrige Phase
8 organische Phase $C_{10}$-Alkohol abgereichert mit homogenem Wasser (beispielsweise mit n= 5 im Wesentlichen Wasser (homogen), Butan, Buten, $C_5$-Aldehyde, 2-Metylbutanol, Pentanol, Nonan, $\alpha,\beta$-ungesättigte $C_{10}$ Aldehyde, $C_{10}$-Aldehyde, $\alpha,\beta$-ungesättigte $C_{10}$-Alkohole)
9 Leichtstrom (beispielsweise mit n= 5 im Wesentlichen Wasser (homogen), Butan, Buten, $C_5$-Aldehyde, 2-Metylbutanol, Pentanol, Nonan)
10 organische Phase mit homogenem Wasser (beispielsweise mit n= 5 im Wesentlichen Wasser (homogen), Butan, Buten, $C_5$-Aldehyde, 2-Metylbutanol, Pentanol, Nonan)
11 wässrige Phase
12 Hochsiederstrom (beispielsweise mit n= 5 im Wesentlichen 2-Metylbutanol, Pentanol, $\alpha,\beta$-ungesättigte $C_{10}$ Aldehyde, $C_{10}$-Aldehyde, $\alpha,\beta$-ungesättigte $C_{10}$-Alkohole)
14 Hochsiederstrom (beispielsweise mit n= 5 im Wesentlichen $\alpha,\beta$-ungesättigte $C_{10}$ Aldehyde, $C_{10}$-Aldehyde, $\alpha,\beta$-ungesättigte $C_{10}$-Alkohole)
15 Hochsiederstrom (beispielsweise mit n= 5 im Wesentlichen $C_{10}$-Alkhohole, C10+- Oligomere mit mehr als 10 Kohlenstoffatomen)
18 Hochsiederstrom (beispielsweise mit n= 5 im Wesentlichen C10+-Oligomere mit der mehr als 10 Kohlenstoffatomen)
19 Rücklauf

**Patentansprüche**

**1.** Verfahren zur Herstellung von gesättigten $C_n$- und $C_{2n}$-Alkoholen mit n = 4, 5 und 6 umfassend die Verfahrensschritte

a) Aldolkondensation isomerer $C_n$-Aldehyde mit n = 4, 5 und 6 unter Erhalt eines Gemisches aus $C_n$- und $\alpha,\beta$-ungesättigten $C_{2n}$-Aldehyden, wobei der Anteil unverzweigter Aldehyde mindestens 20 Gew.-%, bezogen auf die eingesetzten $C_n$-Aldehyde mit n = 4, 5 und 6, beträgt
b) Hydrierung des in a) erhaltenen Gemisches aus $C_n$ und $\alpha,\beta$-ungesättigten $C_{2n}$-Aldehyden mit Wasserstoff unter Erhalt eines Gemisches aus gesättigten $C_n$ und $C_{2n}$-Alkoholen und

c) Auftrennung des Gemisches aus $C_n$ und $C_{2n}$-Alkoholen,

wobei die Auftrennung der $C_n$-Alkohole und der $C_{2n}$-Alkohole durch mindestens ein Zweikolonnensystem oder durch mindestens eine Trennwandkolonne erfolgt, **dadurch gekennzeichnet, dass** das Isomerenverhältnis des erhaltenen $C_n$-Alkohols durch die in Schritt a) durchgeführte Aldolkondensation eingestellt wird, wobei der Anteil an linearen $C_n$-Alkoholen bei mindestens 10 Mol-% und weniger als 60 Mol-%, bezogen auf das Gemisch der isomeren $C_n$-Alkohole, liegt

2. Verfahren gemäß Anspruch 1, **dadurch gekennzeichnet, dass** die in Schritt a) eingesetzten $C_n$-Aldehyde mit n = 4, 5 und 6 durch Hydroformylierung isomerer Olefine mit 3 bis 5 Kohlenstoffatomen mit Synthesegas in Gegenwart eines Hydroformylierungskatalysators zu den entsprechenden unter Bildung der genannten Aldehyden hergestellt wurden.

3. Verfahren gemäß Anspruch 2, **dadurch gekennzeichnet, dass** in der Hydroformylierung ein Katalysatorsystem eingesetzt, das Rhodium als Zentralatom enthält und mit dem Liganden (1) komplexiert ist:

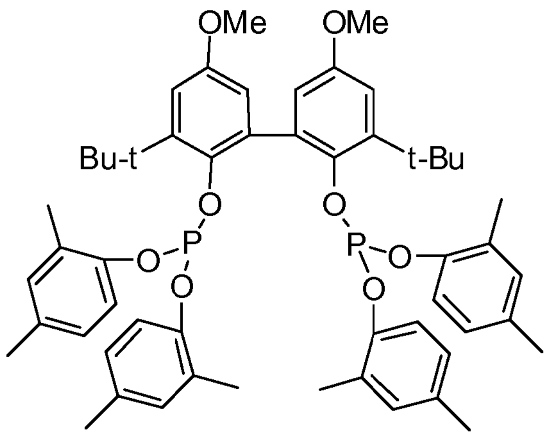

(1).

4. Verfahren gemäß einem oder mehreren der Ansprüche 1 bis 3, **dadurch gekennzeichnet, dass** die Aldolkondensation in Schritt a) in Gegenwart von Natronlauge erfolgt.

5. Verfahren gemäß einem oder mehreren der Ansprüche 1 bis 4, **dadurch gekennzeichnet, dass** die Aldolkondensation der $C_n$-Aldehyde gemäß Schritt a) in einem Rohrreaktor erfolgt, der mindestens ein Mischmodul enthält, das den Edukt-Aldehyd in Tröpfchen mit einem durchschnittlichen Durchmesser (Sauter-Durchmesser) von 0,2 mm bis 2 mm in der kontinuierlichen Katalysatorphase (Prozesslauge) dispergiert, die aus Natronlauge und Natriumsalzen von Carbonsäuren besteht und einen Natriumgehalt von 0,6 bis 1,75 Massen-% sowie einen pH-Wert im Bereich von 12,5 bis 13,5 aufweist.

6. Verfahren gemäß einem oder mehreren der Ansprüche 1 bis 5, **dadurch gekennzeichnet, dass** die Aldolkondensation der $C_n$-Aldehyde gemäß Schritt a) im Temperaturbereich von 100 bis 150 °C durchgeführt wird.

7. Verfahren gemäß einem oder mehreren der Ansprüche 1 bis 6, **dadurch gekennzeichnet, dass** der Reaktionsdruck im Reaktor bei der Aldolkondensation der $C_n$-Aldehyde gemäß Schritt a) im Bereich von 0,2 bis 1,0 MPa liegt.

8. Verfahren gemäß einem oder mehreren der Ansprüche 1 bis 7, **dadurch gekennzeichnet, dass** die Hydrierung gemäß Schritt b) im Temperaturbereich von 170 °C bis 200 °C bei einem Druck von $15*10^5$ Pa bis $30*10^5$ Pa an einem Trägerkatalysator, der als aktive Komponenten zumindest Nickel und Kupfer enthält, erfolgt.

9. Verfahren gemäß einem oder mehreren der Ansprüche 1 bis 8, **dadurch gekennzeichnet, dass** die Hydrierung gemäß Schritt b) mit Wasserstoff in einem Druckbereich von 5 bis 100 bar, durchgeführt und die Hydriertemperaturen zwischen 120 und 220 °C liegen.

10. Verfahren gemäß einem oder mehreren der Ansprüche 1 bis 9, **dadurch gekennzeichnet, dass** n = 5 ist.

## Claims

1. Method for preparing saturated $C_n$- and $C_{2n}$-alcohols where n = 4, 5 and 6 comprising the method steps of

   a) aldol condensation of isomeric $C_n$-aldehydes where n = 4, 5 and 6 to obtain a mixture of $C_n$- and $\alpha$, $\beta$-unsaturated $C_{2n}$-aldehydes, wherein the proportion of unbranched aldehydes is at least 20% by weight, based on the $C_n$-aldehydes used where n = 4, 5 and 6
   b) hydrogenating the mixture of $C_n$ and $\alpha$, $\beta$-unsaturated $C_{2n}$-aldehydes obtained in a) with hydrogen to obtain a mixture of saturated $C_n$ and $C_{2n}$-alcohols and
   c) separating the mixture of $C_n$ and $C_{2n}$-alcohols,

   wherein the separation of the $C_n$-alcohols and the $C_{2n}$-alcohols is effected by means of at least one two-column system or by means of at least one dividing wall column, **characterized in that** the isomer ratio of the $C_n$-alcohol obtained is adjusted by the aldol condensation carried out in step a), wherein the proportion of linear $C_n$-alcohols is at least 10 mol% and less than 60 mol%, based on the mixture of isomeric $C_n$-alcohols.

2. Method according to Claim 1, **characterized in that** the $C_n$-aldehydes where n = 4, 5 and 6 used in step a) were prepared by hydroformylation of isomeric olefins having 3 to 5 carbon atoms with synthesis gas in the presence of a hydroformylation catalyst to the corresponding to form the aldehydes specified.

3. Method according to Claim 2, **characterized in that** in the hydroformylation a catalyst system is used which comprises rhodium as central atom and is complexed with the ligand (1):

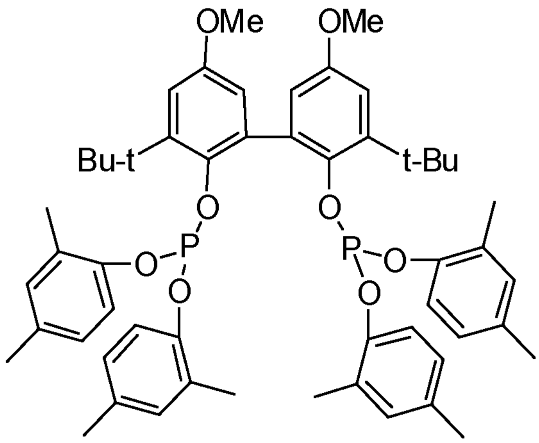

(1) .

4. Method according to one or more of Claims 1 to 3, **characterized in that** the aldol condensation in step a) is carried out in the presence of aqueous sodium hydroxide solution.

5. Method according to one or more of Claims 1 to 4, **characterized in that** the aldol condensation of the $C_n$-aldehydes according to step a) is carried out in a tubular reactor which comprises at least one mixing module which disperses the reactant aldehyde into droplets having an average diameter (Sauter diameter) of 0.2 mm to 2 mm in the continuous catalyst phase (process liquor), which consists of aqueous sodium hydroxide solution and sodium salts of carboxylic acids and has a sodium content of 0.6 to 1.75% by mass and a pH in the range from 12.5 to 13.5.

6. Method according to one or more of Claims 1 to 5, **characterized in that** the aldol condensation of the $C_n$-aldehydes according to step a) is carried out in the temperature range from 100 to 150°C.

7. Method according to one or more of Claims 1 to 6, **characterized in that** the reaction pressure in the reactor during the aldol condensation of the $C_n$-aldehydes according to step a) is in the range from 0.2 to 1.0 MPa.

8. Method according to one or more of Claims 1 to 7, **characterized in that** the hydrogenation according to step b) is carried out in the temperature range from 170°C to 200°C at a pressure of $15*10^5$ Pa to $30*10^5$ Pa over a supported catalyst which contains at least nickel and copper as active components.

9. Method according to one or more of Claims 1 to 8, **characterized in that** the hydrogenation according to step b) is carried out with hydrogen in a pressure range from 5 to 100 bar and the hydrogenation temperatures are between 120 and 220°C.

10. Method according to one or more of Claims 1 to 9, **characterized in that** n = 5.

**Revendications**

1. Procédé pour la préparation d'alcools saturés en $C_n$ et en $C_{2n}$, n = 4, 5 et 6, comprenant les étapes de procédé

a) condensation aldol d'aldéhydes en $C_n$ isomères, n = 4, 5 et 6, avec obtention d'un mélange d'aldéhydes en $C_n$ et d'aldéhydes en $C_{2n}$ $\alpha$, $\beta$-insaturés, la proportion d'aldéhydes non ramifiés représentant au moins 20% en poids par rapport aux aldéhydes en $C_n$ utilisés, n = 4, 5 et 6,
b) hydrogénation du mélange obtenu dans a) d'aldéhydes en $C_n$ et d'aldéhydes en $C_{2n}$ $\alpha$, $\beta$-insaturés avec de l'hydrogène avec obtention d'un mélange d'alcools en $C_n$ et $C_{2n}$ saturés et
c) séparation du mélange d'alcools en $C_n$ et en $C_{2n}$,

la séparation des alcools en $C_n$ et des alcools en $C_{2n}$ ayant lieu par au moins un système à deux colonnes ou par au moins une colonne à paroi de séparation, **caractérisé en ce que** le rapport d'isomères de l'alcool en $C_n$ obtenu est réglé par la condensation aldol réalisée dans l'étape a), la proportion d'alcools en $C_n$ linéaires se situant à au moins 10% en mole et à moins de 60% en mole, par rapport au mélange d'alcools en $C_n$ isomères.

2. Procédé selon la revendication 1, **caractérisé en ce que** les aldéhydes en $C_n$ utilisés dans l'étape a), n = 4, 5 et 6, ont été préparés par hydroformylation d'oléfines isomères comprenant 3 à 5 atomes de carbone avec du gaz de synthèse en présence d'un catalyseur d'hydroformylation au correspondant avec formation des aldéhydes mentionnés.

3. Procédé selon la revendication 2, **caractérisé en ce qu'**un système catalytique, qui contient du rhodium comme atome central et qui est complexé par les ligands (1), est utilisé dans l'hydroformylation :

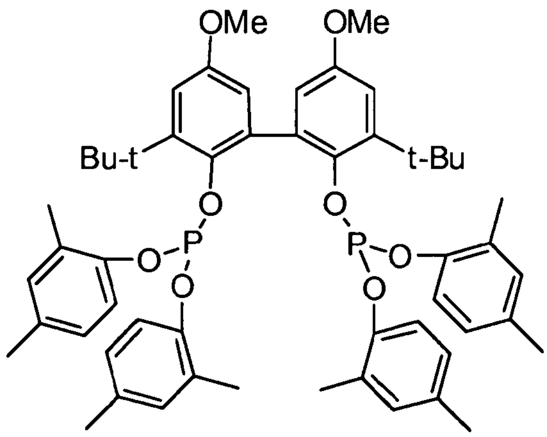

(1).

4. Procédé selon l'une ou plusieurs des revendications 1 à 3, **caractérisé en ce que** la condensation aldol dans l'étape a) a lieu en présence de lessive de soude caustique.

5. Procédé selon l'une ou plusieurs des revendications 1 à 4, **caractérisé en ce que** la condensation aldol des aldéhydes en $C_n$ selon l'étape a) a lieu dans un réacteur tubulaire qui contient au moins un module de mélange qui disperse l'aldéhyde de départ en gouttelettes présentant un diamètre moyen (diamètre de Sauter) de 0,2 mm à 2 mm dans la phase catalytique continue (lessive du procédé), qui est constituée par de la lessive de soude caustique et des sels sodiques d'acides carboxyliques et qui présente une teneur en sodium de 0,6 à 1,75% en masse ainsi qu'un pH dans la plage de 12,5 à 13,5.

6. Procédé selon l'une ou plusieurs des revendications 1 à 5, **caractérisé en ce que** la condensation aldol des aldéhydes en $C_n$ selon l'étape a) est réalisée dans la plage de température de 100 à 150°C.

7. Procédé selon l'une ou plusieurs des revendications 1 à 6, **caractérisé en ce que** la pression de réaction dans le réacteur lors de la condensation aldol des aldéhydes en $C_n$ selon l'étape a) est située dans la plage de 0,2 à 1,0 MPa.

8. Procédé selon l'une ou plusieurs des revendications 1 à 7, **caractérisé en ce que** l'hydrogénation selon l'étape b) a lieu dans la plage de température de 170°C à 200°C à une pression de 15 * $10^5$ Pa à 30 * $10^5$ Pa sur un catalyseur supporté, qui contient comme composants actifs au moins du nickel et du cuivre.

9. Procédé selon l'une ou plusieurs des revendications 1 à 8, **caractérisé en ce que** l'hydrogénation selon l'étape b) est réalisée avec de l'hydrogène dans une plage de pression de 5 à 100 bars et les températures d'hydrogénation se situent entre 120 et 220°C.

10. Procédé selon l'une ou plusieurs des revendications 1 à 9, **caractérisé en ce que** n = 5.

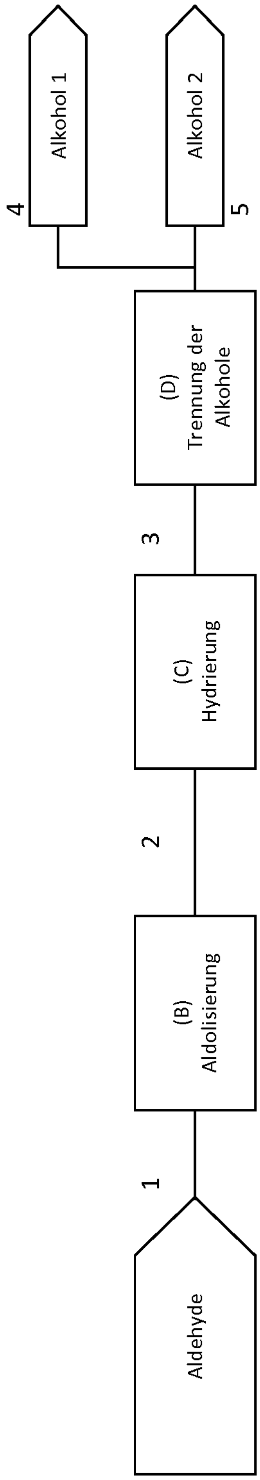
Aldehyde
1
(B)
Aldolisierung
2
(C)
Hydrierung
3
(D)
Trennung der
Alkohole
4
5
Alkohol 1
Alkohol 2

Fig. 1

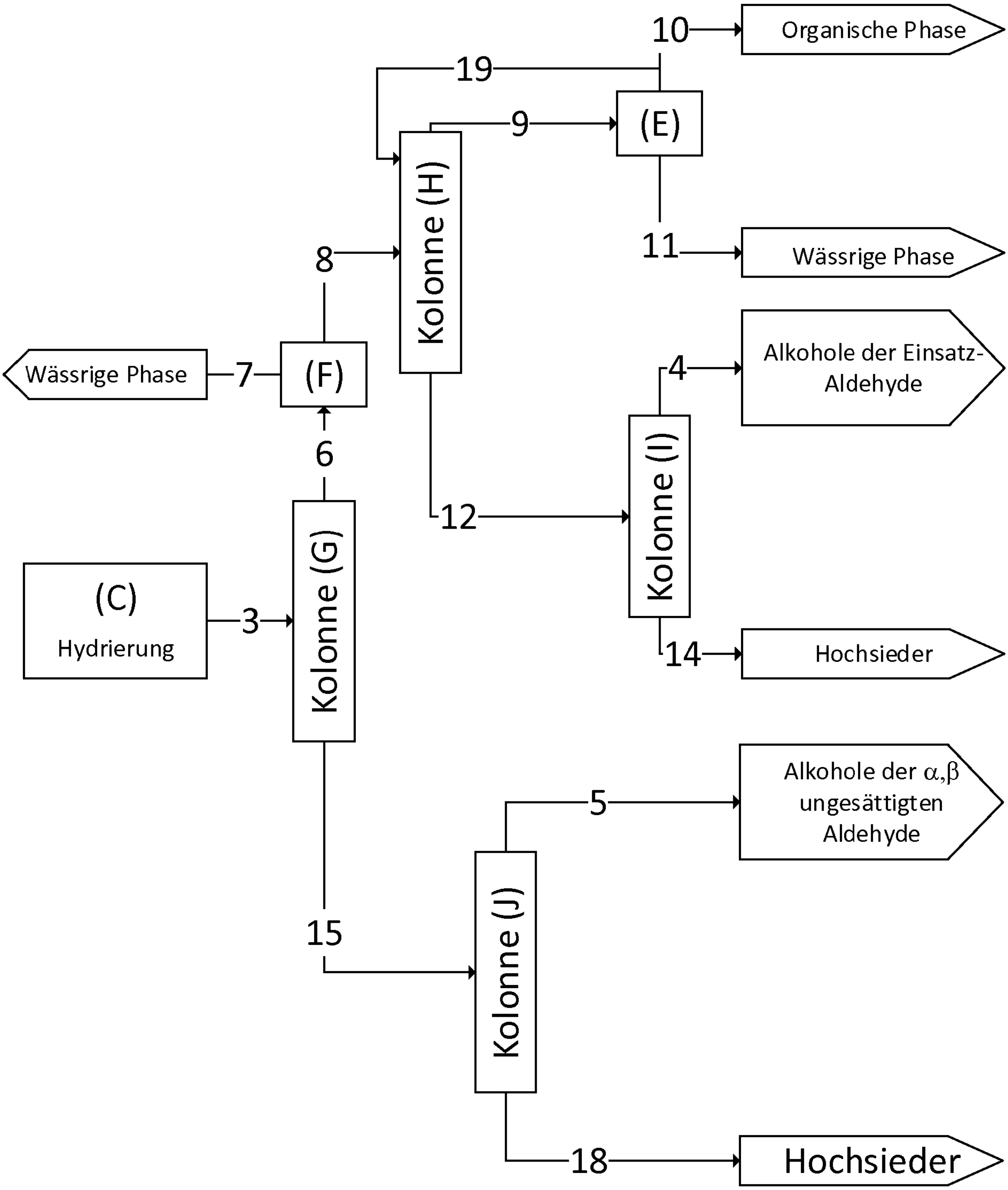

10
Organische Phase
19
9
(E)
Kolonne (H)
11
Wässrige Phase
8
Wässrige Phase
7
(F)
4
Alkohole der Einsatz-Aldehyde
6
Kolonne (I)
12
(C)
Hydrierung
3
Kolonne (G)
14
Hochsieder
5
Alkohole der α,β ungesättigten Aldehyde
15
Kolonne (J)
18
Hochsieder


Fig. 2

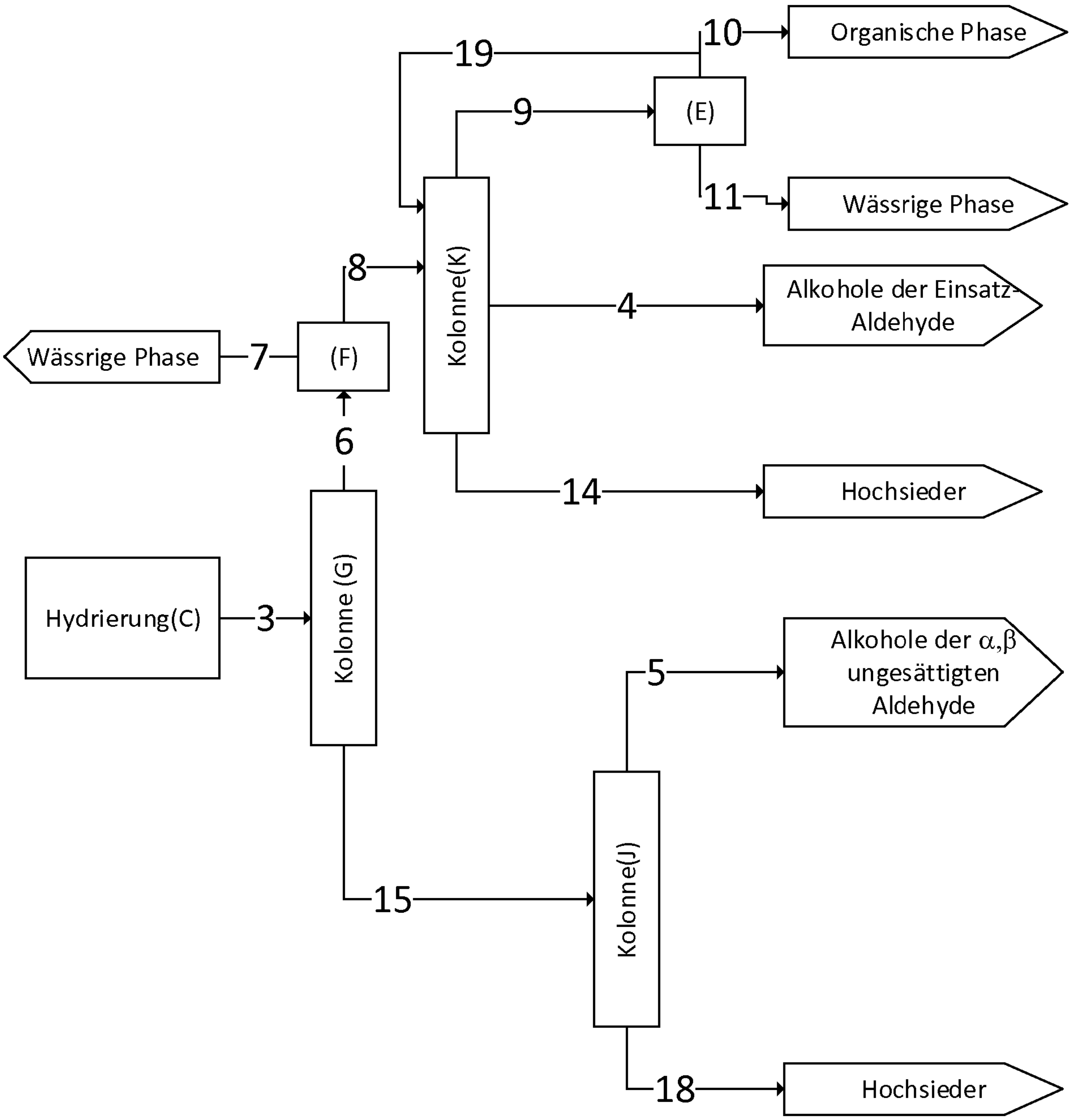
Organische Phase
10
19
9
(E)
11
Wässrige Phase
8
Kolonne(K)
4
Alkohole der Einsatz-Aldehyde
Wässrige Phase
7
(F)
6
14
Hochsieder
Hydrierung(C)
3
Kolonne (G)
5
Alkohole der α,β ungesättigten Aldehyde
15
Kolonne(J)
18
Hochsieder

Fig. 3

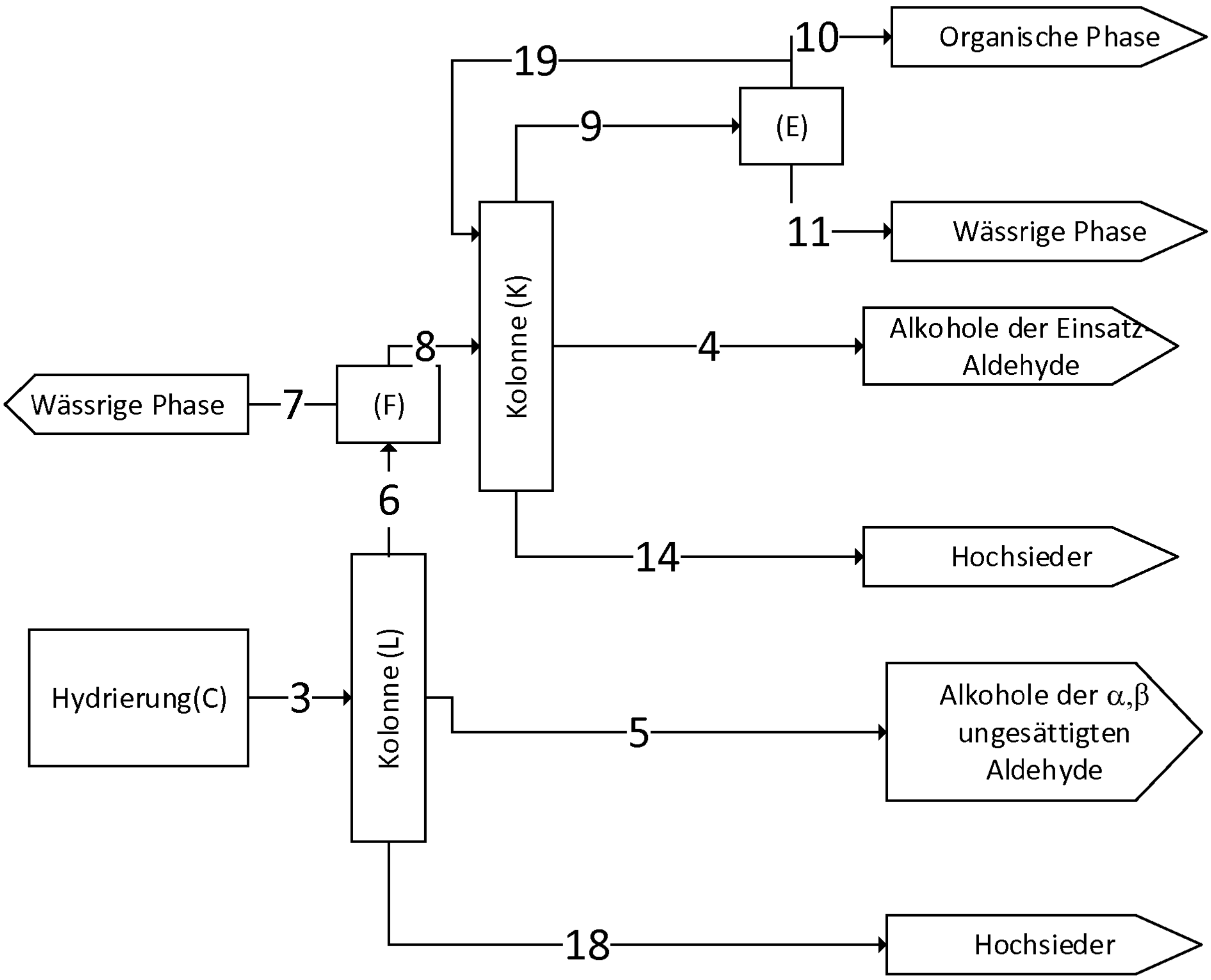

10
Organische Phase
19
9
(E)
11
Wässrige Phase
Kolonne (K)
8
4
Alkohole der Einsatz-Aldehyde
Wässrige Phase
7
(F)
6
14
Hochsieder
Hydrierung(C)
3
Kolonne (L)
5
Alkohole der α,β ungesättigten Aldehyde
18
Hochsieder


Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2014350307 A
- US 2002133047 A
- US 6455743 B
- US 2010048959 A
- DE 4243524
- WO 2017080690 A
- DE 19957522 A1
- DE 102009045139 A1
- DE 102009001594 A1
- DE 19849922
- DE 19849924
- EP 3037400 A
- DE 19933348
- DE 102009045718 A1


### In der Beschreibung aufgeführte Nicht-Patentliteratur


- Hydroformylation Fundaments. **FACHMANN.** Procceses and Applications in Organic Synthesis. Willey VCH Verlag GmbH & Co, vol. 1,2
- **J. FALBE.** New Syntheses with Carbon Monoxide. Springer-Verlag, 1980, 99
- **KIRK-OTHMER.** Encyclopedia of Chemical Technology. John Wiley & Sons, 1996, vol. 17, 902-919